# NEW EUROPEAN PATENT SPECIFICATION

(11) **EP 1 196 122 B2**
(45) Date of publication and mention of the opposition decision: **23.11.2011**
(45) Mention of the grant of the patent: 13.09.2006
(21) Application number: 00927911.8
(22) Date of filing: 20.05.2000
(51) Int. Cl.: A61F 13/15

(54) **FLUID INTAKE INTENSIFIER**
INTENSIVIERUNGSMITTEL ZUR FLÜSSIGKEITSAUFNAHME
INTENSIFICATEUR D'ABSORPTION DE LIQUIDES

(30) Priority: 21.05.1999 KR 9918375
(43) Date of publication of application: 17.04.2002
(73) Proprietor: Yuhan-Kimberly Ltd., Kangnam, Seoul 135-725 (KR)
(72) Inventor: KIM, Doo-Hong, Kunpo-shi, Kyunggi-do 435-040 (KR); KIM, Hyeong Beom, Anyang-shi, Kyunggi-do 431-050 (KR); HWANG, Eo-Yeon, Uwang-shi, Kyunggi-do 437-080 (KR); KANG, Eun-Jung, Dongjack-ku, Seoul 156-060 (KR)
(74) Representative: Mabey, Katherine Frances
(86) International application number: PCT/KR2000/000507
(87) International publication number: WO 2000/071067

(56) References cited:
- EP-B1- 0 773 765
- WO-A1-93/11725
- WO-A1-94/10957
- WO-A1-98/24960
- WO-A1-99/00098
- WO-A2-96/26699
- WO-A2-96/41045
- GB-A- 2 286 967
- GB-A- 2 286 967
- US-A- 4 950 264
- US-A- 5 522 810
- US-A- 5 607 414
- US-A- 5 674 341
- US-A- 5 752 945
- US-A- 5 795 344
- US-A- 5 795 344
- US-A- 5 843 063
- US-A- 5 853 402
- US-A- 5 853 402

## Description

### Field of Invention

The present invention relates generally to sanitary napkins.

### Background of the Invention

Sanitary napkins and incontinence pads (hereinafter also collectively referred to as "pads") are well known in the personal care and hygiene art. Such pads are generally constructed with a liquid pervious top sheet, a liquid impervious back sheet and an absorbent core disposed between the top and back sheets. The liquid pervious top sheet allows menstrual fluids or urine to seep through to the absorbent core which is designed to retain the fluids while the liquid impervious back sheet prevents further seepage.

Problems which have existed and continue to exist in the art are the ability of the liquid pervious top sheet to allow quick seepage (intake) in one direction towards the conventional absorbent core while preventing return in the opposite direction (wet return) thereby reducing discomfort to the user. The rate at which intake occurs determines whether side leakage is reduced or whether fluids are appropriately concentrated in a desired target area.

Over the years many solutions have been proposed. The planar design of sanitary napkins and incontinence pads have been modified in a multitude of ways in an effort to supplement the above-described general depth structure. Pads have been designed to conform in shape to the crotch area so that concave side portions fit the natural curves of the thighs. The reduction of the lateral sides of the planar design in conventional pads resulted in side leakage which soiled underwear and clothing. To address such side leakage, lateral wings and flaps were introduced making pad designs progressively more complicated.

Supplementing planar design of the overall pad were efforts to concentrate menstrual discharge or urine to confined areas. Embossed channels were stamped onto the top sheet and through to the absorbent core to define physical target areas where it was hoped that fluid would concentrate, usually in central areas of the pad. Several shape designs for the embossed areas have been proposed from the straight periphery, rectangular shape to variations such as curved or arcuate peripheries, hour-glass and race-track configurations.

Another direction attempted was the use of a surge. A surge is a strip made of a porous nonwoven material having a planar size usually smaller than that of the absorbent core and used as a composite with the topsheet. Variations to the use of a surge include composites with the entire planar surface of a topsheet. A surge is laminated to the topsheet by thermal, ultrasonic or mechanical bonding, or combinations thereof, to ensure its position in the target area. Thus, a surge is laminated to the target area or laminated onto substantially the entire surface of the liquid pervious topsheet. Conventional surge materials have low porosity and resilience and deteriorate during bonding with the topsheet. Due to these drawbacks, the desired objectives to increase intake rate and reduce wet return have not been achieved.

Another conventional approach is to use an absorbent material such as fiberized pulp in a double layer along the length of the core or concentrated in the central target area. However, this approach provides discomfort to the user especially during activity or movement.

GB 2286967A discloses an absorbent article having a bodyside liner in the form of an apertured film/nonwoven composite.

EP 0773765A1 discloses an absorbent article having an airlaid layer

US 5,853,402 discloses an absorbent core structure for an absorbent article.

All of these efforts have produced less than complete satisfaction and the search continues for a solution to these problems which exist in the art. This discloses presents a low density, lofty, nonwoven pledget for either thick (slim) or thin (ultra-slim) sanitary napkins, having all the advantages of and meet the following objectives.

### Objects of the Invention

Accordingly, it is a general object of the present invention to provide improved sanitary napkins.

### Summary of the Invention

The present invention provides a sanitary napkin in accordance with claim 1.

The sanitary napkin comprises an absorbent core and an intake intensifier pledget located on a central portion of the absorbent core. The pledget is comprised of a first layer and second layer, wherein the first layer is comprised of a TABCW material and the second layer is comprised of an airlaid nonwoven material.

The TABCW material provides a low densified loft through air bonded carded web which has a specific weight basis level at about 15 to about 70 gsm (gram per m²). The TABCW material can comprise about 3 to about 10 denier staple fiber. The TABCW material can also comprise an Ultra Bulky (UB) bicomponent fiber or composites thereof. The airlaid nonwoven material is on about 50 to about 300 gsm (gram per m²) weight basis, and the basis provides for high void volume. The airlaid nonwoven material may include SAM (granule or fiber).

The absorbent core may be a SAP sheet which is a composite of SAM (granule or fiber) and pulp, tissue, a nonwoven material or a mixture of fluff and SAM. The absorbent core may further comprise a tissue-like wrapping or nonwoven material.

The pledget located on the central portion of the absorbent core has a length of at least about 50 mm and a width from about 30 to about 60 mm. The sanitary napkin further comprises a cover sheet, a wrapping material and a baffle portion. The pledget has a first surface situated under the cover sheet and a second surface adhesively bonded to at least one of the absorbent core or the wrapping material. The sanitary napkin can further comprise a fluid distribution layer.

The cover sheet, the wrapping material, the absorbent core and the baffle portion can be compressed in that order to a total width of less than about 1 cm to form a channel around the pledget. The cover sheet is comprised of a liquid pervious material which is adapted to allow menstrual fluids or urine to seep through to the pledget. The baffle portion is comprised of a liquid impervious material adapted to prevent further seepage of menstrual fluids or urine.

In embodiments of the present invention, a combination of a cover sheet comprised of a hydroentangled, hydroapertured spun-lace material and a pledget comprised of a TABCW material is synergistically used to speed up intake time, reduce wet return and enhance retention by the absorbent core. The hydroentangled, hydroapertured spun-lace material can comprise a rayon fiber, and preferably a homogenous mixture of 70% rayon fiber and 30% polyethyleneterephthalate (PET) polyester fiber.

The invention is described in further detail below in conjunction with the figures which are briefly described immediately below.
Fig. 1 is a planar view of one embodiment of a pad according to the present invention in which the cover sheet is made of a transparent material (indicated by an arrow) and overlies the pledget, and the absorbent core (also under the transparent cover sheet) is depicted with phantom lines.
Fig. 2 is an expanded, cross-sectional view of the embodiment shown in Fig. 1 taken along line 2-2.
Fig. 3 is a planar view depicting another embodiment of the pad of the present invention. As in Fig. 1, Fig. 3 shows a cover sheet made of a transparent material and indicated with an arrow. The transparent cover sheet overlies the intensifier strip and the absorbent core (which is delineated by phantom lines).
Fig. 4 is an expanded, cross sectional view of the embodiment shown in Fig. 3 taken along line 4-4.
Fig. 5 is a planar view of a third embodiment of a pad according to the present invention. As in Figs. 1 and 3, the cover sheet in Fig. 5 is also made of a transparent material and indicated with an arrow. The intake intensifier pledget and absorbent core are shown under the transparent cover sheet.
Fig. 6 is an expanded, cross sectional view of the embodiment shown in Fig. 5 taken along line 6-6.

### Detailed Description of the Invention

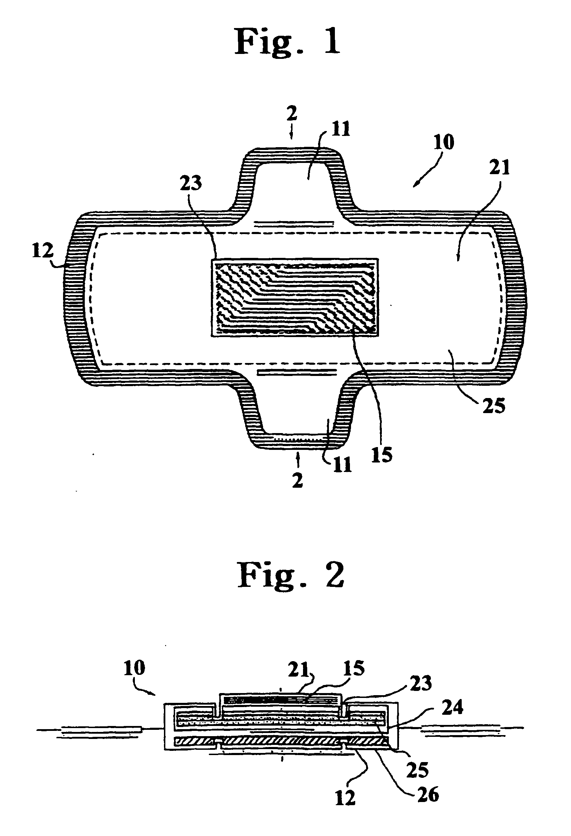

Referring to Fig. 1, the first embodiment of the present invention will now be described. Fig. 1 shows a plan view of a sanitary napkin 10 comprised of wings 11, baffle portion 12, intake intensifier pledget 15, cover sheet 21, optional embossed channel 23 and absorbent core 25. Baffle portion 12 is shown extending along the entire periphery of sanitary napkin 10. Cover sheet 21 is shown transparent and indicated with an arrow and overlies pledget 15 and absorbent core 25 (depicted with phantom lines). Wings 11 can be considered lateral extensions of baffle portion 12 or cover sheet 21. One skilled in the art will appreciate that the present invention does not require the wings as essential features to practice the invention and a modified embodiment without wings is included in the scope of this disclosure.

As shown, located on a central portion (target area) of sanitary napkin 10 is intake intensifier pledget 15, surrounded by optional embossed channel 23. Fig. 1 depicts pledget 15 as having a rectangular shape, but it will be apparent to one skilled in the art that this shape is only provided by way of example and that other shapes such as a square, curved or arcuate peripheries, race-track or hour-glass configurations are all possible while remaining within the scope of this invention after having the benefit of this disclosure. Pledget 15 may also be in two layers, each layer having the same shape or different shape. For optimum target area coverage, it is preferred to have pledget lengths of at least 50 mm and widths of 30 to 60 mm.

Fig. 2 is a cross sectional view taken along line 2-2 in Fig. 1. Fig. 2 is expanded from true scale in order to distinguish the various layers and is in actuality a depiction of a thin pad embodiment. Beginning from the top, Fig. 2 shows sanitary napkin 10 as comprising cover sheet 21, pledget 15, wrapping material 24, absorbent core 25 and baffle portion 12. Fig. 2 also depicts embossed channel 23 and fluid distribution layer 26. Cover sheet 21 and baffle 12 are sealed at their edges to form a sealing margin with various patterns and using various processes such as a mechanical press, heat, adhesive, ultrasonic, combinations thereof and the like.

Wrapping material 24 can include tissue, an air-laid material or a non-woven material. Absorbent core 25 may be a SAP sheet which is a composite of SAM (granule or fiber) and pulp, tissue, a non-woven material, or a mixture of fluff and SAM.

Cover sheet 21 comprises a hydroentangled, hydroapertured spun-lace material. For example, the spun-lace material can comprise a rayon fiber, and preferably, a homogeneous mixture of 70% rayon fiber and 30% PET polyester fiber. In addition, cotton fiber can be used in place of rayon. The ratio of rayon or cotton fiber to PET may vary from 10:0 to 0: 10 and the average mass can range from 20 - 40 gsm.

In terms of its structure, the cover sheet can comprise layers of different fibers. The relative area for hydroaperture can vary depending on the kind of mesh pattern belt used, but should be at least 20% of the total surface area of the cover sheet.

Channel 23 is formed by compressing cover sheet 21, absorbent core 25 and a portion of the baffle 12 to less than I cm width around and spaced apart from pledget 15.

Pledget 15 is comprises a first layer and a second layer, the first layer being comprised of a TABCW material, and the second layer of an airlaid nonwoven material. For the airlaid nonwoven material, it is preferred to use a thermally-bonded airlaid adhesive bonding process on a 50 to 300 gsm (gram per m²) weight basis for high void volume to affix the material to absorbent core 25. Alternatively, a binder-bonded or a multi-bonded air-laid material may be used to bond the material to absorbent core 25. The airlaid material may include SAM. For the TABCW material, a low densified lofty through air bonded carded web is used which has a specific weight basis level at 15 to 70 gsm which comprise a 3 to 6 denier staple fiber, and more preferably, Ultra Bulky (UB) bicomponent fiber or composites thereof.

The airlaid nonwoven material and TABCW have relatively higher void volume capacity for greater absorption than conventional surge materials or cores, making the present invention particularly suitable for thin pads. In addition to greater relative absorption of menstrual fluids, airlaid nonwoven materials and TABCW provide rapid absorption to prevent any unnecessary discomfort to the user by slow seepage from cover sheet 21. The high void volume of the present pledget, in addition to rapid absorption, provides greater retention of menstrual fluids to avoid wet return. A combination of top TABCW and bottom air-laid is used to optimize its function when combined together.

Unlike conventional methods of using double layers of core materials or the surge layer laminated to the cover sheet, the present invention introduces the use of airlaid nonwoven materials for pledget 15 to provide a more zoned, area concentrated and intensified fluid intake which prevents lateral run-off and leakage. The low densified nonwoven materials used also provide a more cushioned feel due to their resiliency while being less physically bulky in a topographically limited zone.

Wrapping material 24 is comprised of cellulose tissue, air-laid or a non-woven material and its function is to prevent possible migration of the absorbent SAP sheet material of core 25. Absorbent core 25 is of a SAP sheet material comprised of a densified or undensified, non-woven, super absorbent material (SAM), a non-woven tissue or pulp material, or combinations thereof. Wrapping material 24 can be in an E-fold or C-fold configuration.

The optional fluid distribution layer 26 is comprised of non-woven material of pulp fibers and its function is to help distributing menstrual fluids-keeping integrity.

Baffle portion 12 is comprised of film, a non-woven material, a combination of a film and a non-woven material, other materials of inorganic compounds or combinations thereof, and functions as a liquid impervious back sheet for preventing further seepage.

It has also been unexpectedly discovered that a combination of a cover sheet comprised of a hydroentangled, hydroapertured spun-lace material and a pledget comprised of a TABCW material used synergistically, increases the rate of intake of fluids in the direction towards the pledget and absorbent core. This increased rate enhances the prevention of wet return, concentrates the fluid in the target area and thereby reduces discomfort to the user.

### Example

This example shows tests for a pledget according to the present invention with various degrees of additional absorbent materials selected from TABCW material (T), two airlaid materials (A and A') or combinations thereof, in various further combinations with a cover sheet (C) made of a hydroentangled, hydroapertured spun-lace material.

Specifically, the TABCW material used had an average mass of 25 gsm, airlaid material 1 (A) had an average mass of 130 gsm and a density of 0.09 g/cc, and airlaid material 2 (A') had an average mass of 175 gsm and a density of 0.08 g/cc. The cover sheet comprised a homogeneous mixture of 70% rayon fiber and 30 % PET polyester fiber with an average mass 30 gsm, and each hydroaperture was in a diamond shape made into a mesh pattern by hydro-entanglement. The total surface area for the hydroapertures was approximately 25.1%.

A pad constructed with a pledget according to the present invention and a cover sheet having the materials identified above has a body-side liner which provides a cotton-like appearance and feel with the least possible lint, and which achieves superior intake rate and reduces wet return. For the tests, the following protocols were utilized.
1. Material caliper for thickness measurements. The caliper of a material is a measure of its thickness, and is measured at 345 Pa (0.05 psi) with a Starret-type bulk tester in millimeter (mm) units. In practice, 10 repetitions of any measurement should be made.
2. Rate block intake test. This test is used to determine the intake time of a known quantity of fluid into a material and/or material system. The test apparatus consists of a rate block, a funnel and a timer or stop watch. A 4 X 4 inch (102 mm²) piece of a pledget and/or comparative absorbent materials, and a cover sheet were cut for each test. The sample cover sheet was placed over the pledget and/or comparative absorbent materials to be tested and the rate block was placed on top of the two materials.
   For the tests, 2 ml each of an artificial menses fluid was prepared and delivered into the test apparatus funnel and the timer was initiated. The fluid moved from the funnel into a capillary where it was delivered to the material system. The timer was stopped when all the fluid was absorbed into the material system as observed from the chamber in the test apparatus. The intake time for a known quantity of test fluid was recorded for a given material system. This value is a measure of a materials system's absorbency, with lower intake time representing a more absorbent system. Five to ten repetitions were performed to determine average intake time.
3. Rewet test for wet return. This test is used to determine the amount of fluid that will come back to the surface of a cover sheet when a load is applied. The amount of fluid that comes back through the surface is called the rewet value. If more fluid comes back to the surface, the rewet value is larger, while smaller amounts of fluid return results in lower rewet values. Lower rewet values are associated with a dryer material and hence a dryer pad. When considering rewet, three properties are important -- (a) intake, if the material/system does not have a good intake speed, the fluid has a greater tendency to rewet; (b) fluid hold, the more the absorbent holds onto the fluid the less is available for rewet; and (c) flow-back, the more the cover prohibits fluid from coming back through, the lower the rewet.
   In the testing, 2 ml of artificial menses fluid was introduced into the rate block apparatus and allowed to absorb into a 4" x 4" sample of the cover material which was placed on top of a 4" x 4" pledget and/or comparative absorbent materials. The fluid was allowed to interact with the system for 1 minute with the rate block resting on top of the materials. The material system was placed onto a closed bag, partially filled with a saline solution. The fluid back was positioned on top of a lab jack. Pieces of blotter paper were weighed and placed on top of the material system. The bag with the material system was raised against a fixed acrylic plate using the lab jack until a total of 6895 Pa (1 psi) was applied.
   The pressure was held and fixed for 3 minutes after which it was released and the blotter paper weighed. The blotter paper should have retained any fluid that was transferred to it from the cover/absorbent system. The difference in weight between the original blotter and the blotter after the absorption experiment is the rewet value.
4. Intake and staining test. An intake/staining test enables observations for stain size, intensity and fluid retention in components to be observed with fluid flow rate and pressure.

A material system measuring 4" x 4" was placed beneath an acrylic plate having a 1/8" (3mm) diameter hole bored into the center. A piece of 1/8" tubing was connected to the hole with a fitting. Artificial menses fluid was delivered to the sample using a syringe pump at a specified rate and for a specified volume. In these experiments, the pump was programmed to deliver a total volume of 1 ml to the samples which were under pressures of between 0 Pa and 621 Pa (0.00 psi and 0.09 Psi). These pressures were applied using a weight which was placed on top of the acrylic plates and distributed evenly. The flow rate of the pump was programmed to deliver at a rate of 1 ml/sec.

The stain size area for the tested cover sheets was measured manually and the amount of fluid in each component of the system was measured by weight before and after absorption of the fluid. Fluid retention was measured by weighing the cover sheet before and after fluid introduction. Average stain size fluid retention were determined from at least five repetitions at each pressure.

The following tabulates the results and the abbreviations in the tables are as follows.
CA = Cover-Airlaid 1
CTA = Cover-TABCW-Airlaid 1
CAA' = Cover-Airlaid 1-Airlaid 2
CTAA' = Cover-TABCW-Airlaid 1-Airlaid 2

**Table 1 - Intake**

| | Time (sec.) | Rewet (gm) |
|---|---|---|
| CA | 26.29 | 0.85 |
| CTA | 8.06 | 0.80 |
| CAA' | 9.29 | 0.47 |
| CTAA' | 5.23 | 0.51 |

**Table 2 - Retention - no load**

| | Retain (gm) | Area (mm²) |
|---|---|---|
| CA | 0.28 | 1785 |
| CTA | 0.20 | 1326 |
| CAA' | 0.17 | 1086 |
| CTAA' | 0.19 | 1156 |

**Table 3 - Retention - 0.09 psi**

| | Retain (gm) | Area (mm²) |
|---|---|---|
| CA | 0.39 | 2587 |
| CTA | 0.24 | 1688 |
| CAA' | not tested | not tested |
| CTAA' | 0.21 | 1432 |

As the above results show, the use of a TABCW pledget significantly reduces intake time and increasing absorbent capacity significantly reduces wet return (Rewet). When absorbent capacity is low, use of a TABCW pledget can help reduce the retention load for the cover sheet and reduce the stain size.

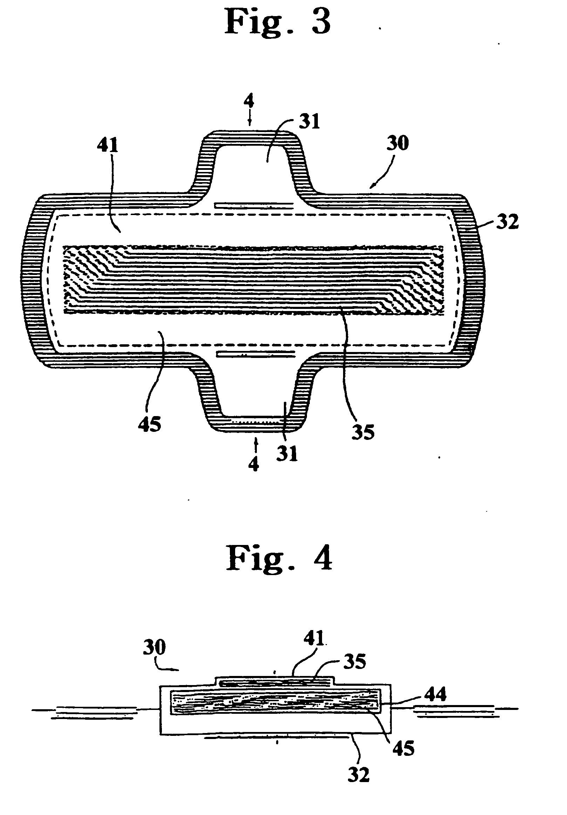

With reference to Fig. 3, a second embodiment of the present invention is described. Fig. 3 is a plan view of sanitary napkin 30 comprised of wings 31, baffle portion 32, intensifier strip 35, cover sheet 41 and absorbent core 45. Baffle portion 32 is shown extending along the entire periphery of sanitary napkin 30. Cover sheet 41 is shown transparent and indicated with an arrow and overlies strip 35 and absorbent core 45 (depicted with phantom lines). As stated above for the first embodiment, the second embodiment may be practiced with equal efficacy as far as the function of the intensifier strip is concerned, with a pad having no wings. Located in a midline portion which includes the target area of sanitary napkin 30 is intake intensifier strip 35. Fig. 3 depicts strip 35 as being in a rectangular shape, but other geometric shapes such as an hour-glass configuration are possible. For optimum target area coverage the length of strip 35, shown in Fig. 3, is enlarged relative to pledget 15 shown in Fig. 1.

Either pledget 15 shown in Fig. 1 or strip 35 shown in Fig. 3, can also be "expanded" to span the entire surface area over absorbent cores 25 (Fig. 1) or 45 (Fig. 3), respectively. To depict this further aspect of the present invention, absorbent cores 25 (Fig. 1) and 45 (Fig. 3) were delineated with phantom lines to demonstrate this capability. In this aspect, one skilled in the art will recognize that the concept of "pledget" or "strip" become merged as they are extended to span the entire surface area over the absorbent cores.

Fig. 4 is a cross sectional view taken along line 4-4 in Fig. 3. As in Fig. 2 (above), Fig. 4 is also an expanded depiction from true scale in order to distinguish the various layers and is also, in actuality, a depiction of another thin pad embodiment. The layers shown in Fig. 4 can be compressed to a total width of less than 1 cm to form a channel around intake intensifier strip 35 if desired.

Starting from the top, Fig. 4 shows sanitary napkin 30 as comprising cover sheet 41, strip 35, wrapping material 44, absorbent core 45, optional fluid distribution layer 46 and baffle portion 32. Cover sheet 41 and baffle portion 32 are sealed at their edges to form a sealing margin with various patterns and using various processes as noted above.

The materials used for strip 35 (and the other components) are identical to those described for the first embodiment, above, and have equal efficacy in practice in terms of the selected materials.

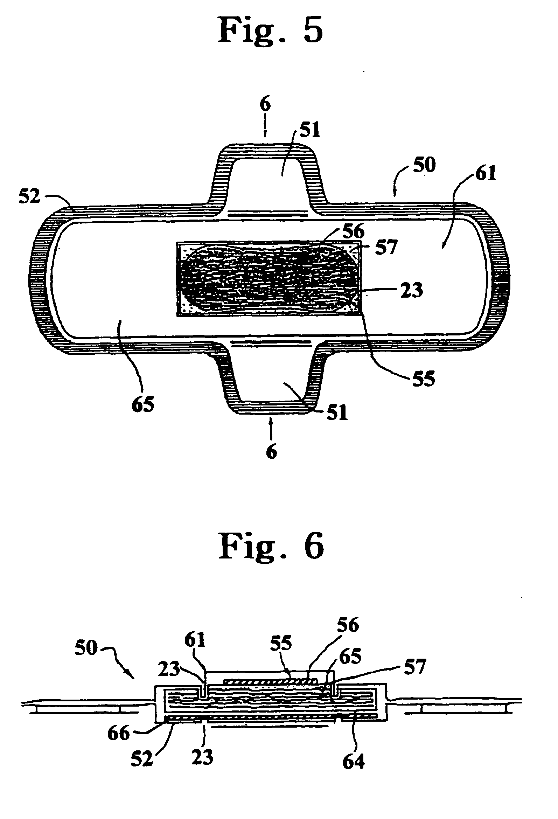

Fig. 5 shows a plan view of sanitary napkin 50 which is a third, thick pad embodiment of the present invention, and which is comprised of wings 51, baffle portion 52, intake intensifier pledget 55, cover sheet 61 and absorbent core 65. Baffle portion 52 is shown extending along the entire periphery of sanitary napkin 50. Cover sheet 61 is shown transparent and indicated with an arrow and intensifier pledget 55 and absorbent core 65.

Situated on a central position encompassing the optimal target area of sanitary napkin 50 is intake intensifier pledget 55- Fig. 5 shows an hour glass shaped pledget 55. For optimum target area coverage, pledget 55 is shaped to conform to the vaginal area as shown. Various other shapes such as a rectangular or racetrack pattern are contemplated as being within the scope of this disclosure.

Fig. 6 is a cross sectional view taken from line 6-6 in Fig. 5. Fig. 6 depicts a thick pad cross-section, but one skilled in the art will appreciate that the pledget 55 (Fig. 5) may be made of airlaid and TABCW as a thin pad.

Fig. 6 shows sanitary napkin 50 as comprising cover sheet 61, pledget 55, wrapping material 64, absorbent core 65 and baffle portion 52. As with the second embodiment, the materials used for pledget 50 and the other components are identical to those described for the first embodiment. Absorbent core 65 may be a SAP sheet or may be substituted with a mixture of fiberized pulp (fluff) and SAM. Absorbent core 65 may be comprised of two layers, a top layer made of unwrapped fluff and a bottom layer made of wrapped or unwrapped fluff and SAM mixture. Alternatively, the SAM mixture can be located between the two layers. Pad 50 may also optionally include a distribution or retention layer 66.

Additionally, Fig. 6 shows a variation to pledget 55. One skilled in the art will appreciate that such a variation is equally applicable to the intake intensifier pledget 15 shown in Fig. 2 and the intake intensifier strip 35 depicted in Fig. 4 after the following disclosure. Pledget 55 is shown, by way of example, as being comprised of a first layer 56 (in an hour-glass configuration) and a second layer 57 (in a rectangular configuration). First layer 56 is comprised of a TABCW material and second layer 57 is comprised of an airlaid nonwoven material.

Various modifications and alterations to the present invention may be appreciated based on a review of this disclosure. These changes and additions are intended to be within the scope of this invention as defined by the following claims.

## Claims

1. A sanitary napkin (10), comprising:
an absorbent core (25) and an intake intensifier pledget (15) located on a central portion of said absorbent core (25),
**characterized in that** said pledget (15) is comprised of a first layer and a second layer, said first layer being comprised of a Thru Air Bonded Carded Web material and the second layer being comprised of an airlaid nonwoven material,
wherein said Thru Air Bonded Carded Web material provides a low densified lofty through air bonded carded web which has a specific weight basis level at 15 to 70 gsm (gram per m²), and
the sanitary napkin (10) further comprising:
a cover sheet (21), a wrapping material and a baffle portion (12), wherein said pledget has a first surface situated under said cover sheet (21) and a second surface adhesively bonded to at least one of said absorbent core (25) or said wrapping material, and
wherein said cover sheet further comprises a hydroentangled, hydroapertured spun-lace material.

2. The sanitary napkin (10) of claim 1, wherein said airlaid nonwoven material is on 50 to 300 gsm (gram per m²) weight basis, said basis providing for high void volume.

3. The sanitary napkin (10) of any preceding claim, wherein said airlaid nonwoven material includes Superabsorbent Material (granule or fiber).

4. The sanitary napkin (10) of claim 1, wherein said Thru Air Bonded Carded Web material comprises of 3 to 10 denier staple fiber.

5. The sanitary napkin (10) of claim 1, wherein said Thru Air Bonded Carded Web material comprises an Ultra Bulky (UB) bicomponent fiber or composites thereof.

6. The sanitary napkin (10) of any preceding claim, wherein said absorbent core is made of a material selected from the group consisting of a Superabsorbent Polymer sheet, a composite of Superabsorbent Material (granule or fiber) and pulp, a tissue, a nonwoven material and a mixture of fluff and Superabsorbent Material.

7. The sanitary napkin (10) of any preceding claim, wherein said pledget located on said central portion of sad absorbent core (25), has a length of at least 50 mm and a width from 30 to 60 mm.

8. The sanitary napkin (10) of claim 1, further comprising a fluid distribution layer.

9. The sanitary napkin (10) of claim 1, wherein said cover sheet is comprised of a liquid pervious material which is adapted to allow menstrual fluids to seep through to said pledget.

10. The sanitary napkin (10) of claim 1, wherein said baffle portion is comprised of a liquid impervious material adapted to prevent further seepage of menstrual fluids.

11. The sanitary napkin (10) of claim 1, wherein said hydroentangled, hydroapertured spun-lace material is rayon fiber.

12. The sanitary napkin of claim 1, wherein said hydroentangled, hydroapertured spun-lace material is a homogeneous mixture of 70% rayon fiber and 30% polyethylene polyester.

13. The sanitary napkin of any preceding claim, wherein the pledget comprises first and second layers, each layer having the same shape.

14. The sanitary napkin of any preceding claim wherein the airlaid nonwoven material of the pledget is affixed in a thermally-bonded airlaid adhesive bonding process on a 50 to 300 gsm weight basis to the absorbent core (25), or wherein the airlaid material is binder bonded or multibonded to the absorbent core (25).

15. The sanitary napkin of any preceding claim, wherein the pledget is shorter and narrower than the absorbent.

## Patentansprüche

1. Binde (10), welche umfasst:
einen absorbtionsfähigen Kern (25) und einen Aufnahme-Intensivierer-Bausch (15), der an einem zentralen Bereich des absorbtionsfähigen Kerns (25) angeordnet ist,
**dadurch gekennzeichnet, dass** der Bausch (15) eine erste Schicht und eine zweite Schicht umfasst, wobei die erste Schicht ein mit Luft gebundenes, kardiertes Bahnmaterial (Thru Air Bonded Carded Web Material) umfasst und dass die zweite Schicht ein luftgelegtes Vliesmaterial umfasst,
wobei das mit Luft gebundene, kardierte Bahnmaterial (Thru Air Bonded Carded Web Material) eine gering verdichtete, voluminöse, durch Luft gebundene, kardierte Bahn bereitstellt, die ein Niveau einer spezifischen Flächenmasse von 15 bis 70 gsm (Gramm pro m²) aufweist,
wobei die Binde (10) des Weiteren eine Abdeckschicht (21), ein Einhüllmaterial und einen Ableitbereich (12) umfasst, wobei der Bausch eine erste Oberfläche aufweist, die unter der Abdeckschicht (21) liegt, und eine zweite Oberfläche aufweist, die haftend an zumindest entweder dem absorptionsfähigen Kern (25) oder dem Einhüllmaterial gebunden ist, und
wobei die Abdeckschicht des Weiteren ein hydroverfestigtes, hydrogelöchertes Spunlace-Material aufweist.

2. Binde (10) nach Anspruch 1, wobei das luftgelegte Vliesmaterial eine Flächenmasse von 50 bis 300 gsm (Gramm pro m²) aufweist, wobei diese Masse ein hohes Leervolumen bereitstellt.

3. Binde (10) nach einem vorhergehenden Anspruch, wobei das luftgelegte Vliesmaterial ein superabsorbtionsfähiges Material (Granulat oder Faser) umfasst.

4. Binde (10) nach Anspruch 1, wobei das mit Luft gebundene, kardierte Bahnmaterial (Thru Air Bonded Carded Web Material) eine Stapelfaser mit 3 bis 10 Denier aufweist.

5. Binde (10) nach Anspruch 1, wobei das mit Luft gebundene, kardierte Bahnmaterial (Thru Air Bonded Carded Web Material) eine ultra-massige (Ultra Bulky - UB) Biokomponentenfaser oder Verbundstoffe davon aufweist.

6. Binde (10) nach einem vorhergehenden Anspruch, wobei der absorptionsfähige Kern aus einem Material gebildet ist, das ausgewählt ist aus der Gruppe bestehend aus einer Schicht aus superabsorbtionsfähigem Polymer, einem Verbundstoff aus superabsorbtionsfähigem Material (Granulat oder Faser) und Pulpe, einem Papiertuch, einem Vliesmaterial und einer Mischung aus Fluffzellstoff und superabsorbtionsfähigem Material.

7. Binde (10) nach einem vorhergehenden Anspruch, wobei der Bausch, der an dem zentralen Bereich des absorptionsfähigen Kerns (25) angeordnet ist, eine Länge von mindestens 50 mm und eine Breite von 30 bis 60 mm aufweist.

8. Binde (10) nach Anspruch 1, die des Weiteren eine Flüssigkeitsverteilungsschicht aufweist.

9. Binde (10) nach Anspruch 1, wobei die Abdeckschicht ein flüssigkeitsdurchlässiges Material aufweist, das dazu geeignet ist, Menstruationsflüssigkeiten zu dem Bausch hindurchzulassen.

10. Binde (10) nach Anspruch 1, wobei der Ableitbereich ein flüssigkeitsundurchlässiges Material aufweist, das dazu geeignet ist, ein weiteres Hindurchsickern der Menstruationsflüssigkeiten zu verhindern.

11. Binde (10) nach Anspruch 1, wobei das hydroverfestigte, hydrogelöcherte Spunlace-Material eine Rayon-Faser ist.

12. Binde (10) nach Anspruch 1, wobei das hydroverfestigte, hydrogelöcherte Spunlace-Material eine homogene Mischung aus 70% Rayon-Faser und 30% Polyethylenpolyester ist.

13. Binde nach einem vorhergehenden Anspruch, wobei der Bausch eine erste und eine zweite Schicht aufweist, wobei jede Schicht dieselbe Form aufweist.

14. Binde nach einem vorhergehenden Anspruch, wobei das luftgelegte Vliesmaterial des Bauschs in einem thermisch gebundenen Luftlege-Haftbindungsvorgang mit einer Flächenmasse von 50 bis 300 gsm an dem absorptionsfähigen Kern (25) befestigt ist, oder wobei das luftgelegte Material mittels Haftmittel an dem absorptionsfähigen Kern (25) gebunden oder mehrfach gebunden ist.

15. Binde nach einem vorhergehenden Anspruch, wobei der Bausch kürzer und schmaler als der absorptionsfähige Kern ist.

## Revendications

1. Serviette hygiénique (10), comprenant:
un noyau absorbant (25) et un tampon intensificateur d'absorption (15) situé sur une partie centrale dudit noyau absorbant (25),
**caractérisée en ce que** ledit tampon (15) est constitué d'une première couche et d'une deuxième couche, ladite première couche étant constituée d'une matière en bande cardée collée laissant passer l'air, et la deuxième couche étant constituée d'une matière non tissée à couche appliquée par jet d'air, ladite matière en bande cardée collée laissant passer l'air fournissant une bande cardée collée laissant passer l'air élastique faiblement densifiée présentant un niveau de base pondérale spécifique de 15 à 70 gsm (grammes par m²), et la serviette hygiénique (10) comprenant en outre:
un film de recouvrement (21), une matière d'enrobage et une partie de chicane (12), ledit tampon présentant une première surface située en dessous dudit film de recouvrement (21) et une deuxième surface collée de façon adhésive sur au moins soit ledit noyau absorbant (25), soit ladite matière d'enrobage, et ledit film de recouvrement contenant en outre une matière hydro-entremêlée hydro-ajourée lacée par filage.

2. Serviette hygiénique (10) selon la revendication 1, dans laquelle ladite matière non tissée à couche appliquée par jet d'air présente une base pondérale de 50 à 300 gsm (grammes par m²), ladite base fournissant un volume de vides élevé.

3. Serviette hygiénique (10) selon l'une quelconque des revendications précédentes, dans laquelle ladite matière non tissée à couche appliquée par jet d'air comprend une matière super-absorbante (granulaire ou fibreuse).

4. Serviette hygiénique (10) selon la revendication 1, dans laquelle ladite matière en bande cardée collée laissant passer l'air contient des fibres discontinues de 3 à 10 deniers.

5. Serviette hygiénique (10) selon la revendication 1, dans laquelle ladite matière en bande cardée collée laissant passer l'air contient une fibre à deux composants ultra gonflante (UB), ou des composites de celle-ci.

6. Serviette hygiénique (10) selon l'une quelconque des revendications précédentes, dans laquelle ledit noyau absorbant est constitué d'une matière sélectionnée dans le groupe comprenant une feuille de polymère super-absorbant, un composite d'une matière super-absorbante (granulaire ou fibreuse) et de pâte, une ouate, une matière non tissée et un mélange de peluche et de matière super-absorbante.

7. Serviette hygiénique (10) selon l'une quelconque des revendications précédentes, dans laquelle ledit tampon situé sur ladite partie centrale dudit noyau absorbant (25) a une longueur d'au moins 50 mm et une largeur comprise entre 30 et 60 mm.

8. Serviette hygiénique (10) selon la revendication 1, comprenant en outre une couche de répartition de fluide.

9. Serviette hygiénique (10) selon la revendication 1, dans laquelle ledit film de recouvrement est constitué d'une matière imperméable aux liquides adaptée pour permettre aux fluides menstruels de s'infiltrer à travers ledit tampon.

10. Serviette hygiénique (10) selon la revendication 1, dans laquelle ladite partie de chicane est constituée d'une matière imperméable aux liquides adaptée pour empêcher toute fuite supplémentaire de fluides menstruels.

11. Serviette hygiénique (10) selon la revendication 1, dans laquelle ladite matière hydro-entremêlée hydro-ajourée lacée par filage est la fibre de rayonne.

12. Serviette hygiénique selon la revendication 1, dans laquelle ladite matière hydro-entremêlée hydro-ajourée lacée par filage est un mélange homogène de 70 % de fibre de rayonne et de 30 % de polyéthylène polyester.

13. Serviette hygiénique selon l'une quelconque des revendications précédentes, dans laquelle le tampon comprend des première et deuxième couches, chaque couche ayant la même forme.

14. Serviette hygiénique selon l'une quelconque des revendications précédentes, dans laquelle la matière non tissée à couche appliquée par jet d'air du tampon est fixée par un procédé de collage adhésif à couche appliquée par jet d'air à liaison thermique sur une base pondérale de 50 à 300 gsm sur le noyau absorbant (25), ou dans laquelle la matière à couche appliquée par jet d'air est collée par un liant ou est multi-collée sur le noyau absorbant (25).

15. Serviette hygiénique selon l'une quelconque des revendications précédentes, dans laquelle le tampon est plus court et plus étroit que le noyau absorbant.
